(19) Europäisches Patentamt
European Patent Office
Office européen des brevets

(11) Veröffentlichungsnummer: **0 124 561**
**B1**

(12) # EUROPÄISCHE PATENTSCHRIFT

(45) Veröffentlichungstag der Patentschrift: **04.01.89**

(51) Int. Cl.⁴: **C 07 H 21/00**

(21) Anmeldenummer: **83903507.8**

(22) Anmeldetag: **27.10.83**

(86) Internationale Anmeldenummer: **PCT/EP 83/00280**

(87) Internationale Veröffentlichungsnummer: **WO 84/01778 (10.05.84 Gazette 84/12)**

(54) **VERFAHREN ZUR HERSTELLUNG VON OLIGONUCLEOSIDPHOSPHONATEN.**

(30) Priorität: **28.10.82 DE 3239888**

(43) Veröffentlichungstag der Anmeldung: **14.11.84 Patentblatt 84/46**

(45) Bekanntmachung des Hinweises auf die Patenterteilung: **04.01.89 Patentblatt 89/1**

(84) Benannte Vertragsstaaten: **AT BE CH DE FR GB LI LU NL SE**

(56) Entgegenhaltungen:
**Biochemistry 18, No. 23 (1979), 5134-5143**
**CHEMICAL REVIEWS, Band 77, Nr. 2, März 1977 (US) V. Amarnath et al.: "Chemical Synthesis of Oligonucleotides" Seiten 183-217, siehe Seite 210, Tabelle III 16/21**
**NUCLEIC ACIDS RESEARCH, Band 10, Nr. 21, November 1982, IRL Press Ltd. OXFORD (GB) Eiko Ohtsuka et al.: "Recent developments in the chemical synthesis of polynucleotides", Seiten 6553-6570, siehe Seiten 6566-6567**

**Die Akte enthält technische Angaben, die nach dem Eingang der Anmeldung eingereicht wurden und die nicht in dieser Patentschrift enthalten sind.**

(73) Patentinhaber: **BIOSYNTECH Biochemische Synthesetechnik GmbH, Stresemannstrasse 268-280, D-2000 Hamburg 50 (DE)**

(72) Erfinder: **KÖSTER, Hubert, Hallerstrasse 74, D-2000 Hamburg 13 (DE)**

(74) Vertreter: **Henkel, Feiler, Hänzel & Partner, Möhlstrasse 37, D-8000 München 80 (DE)**

**EP 0 124 561 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von Oligonucleosidphosphonaten. Die erfindungsgemäß hergestellten Verbindungen, zu denen auch die entsprechenden Oligodeoxynucleosidphosphonate gehören, können als lipophile Hybridisierungsanaloga eingesetzt werden, d. h. sie können in hydrophile Oligonucleotide eingebaut werden, damit diese die Zellmembran leichter passieren können und in der Zelle mit basenkomplementären Nucleinsäuresequenzen interagieren (hybridisieren).

Nucleinsäureanaloge mit Modifikationen entweder an dem Zuckerteil oder an den Internucleotidbindungen sind bekannt, z. B. in Form von Nucleosidphosphiten, Nucleosidphosphonaten und Nucleosidthiophosphonaten. Auch Deoxynucleosid-methylphosphonatdiester sind bereits hergestellt und untersucht worden. Die Phenyl- und Methylphosphonodiester von Deoxynucleosiden sind unter Verwendung einer modifizierten Triestermethode in homogener Phase erhalten worden, allerdings bei längeren Kondensationszeiten; für die Herstellung der Produkte in reiner Form sind umfangreiche Reinigungsmaßnahmen erforderlich gewesen.

Es sind Oligonucleotidsynthesen bekannt, die rasch durchgeführt werden können und hinreichend gute Ausbeuten liefern, bei denen ein Teil der Reaktanten chemisch an einen polymeren Träger, z. B. Glas mit kontrollierten Porendurchmessern, gebunden sind. So wird beispielsweise in "Nucleic Acids Research", Vol. 10, No. 21, November 1982, insbesondere Seiten 6564/6565 unter Bezugnahme auf Originalliteratur berichtet, daß Kieselgel bei der Deoxynucleotidsynthese nach der Phosphit- oder Triestermethode einen geeigneten Träger darstellt. Das Verfahren der Erfindung verwendet ebenfalls polymer gebundene Reaktanten; es kann in extrem kurzer Zeit durchgeführt werden und liefert hohe Ausbeuten der gewünschten Nucleosidphosphonate.

Die erfindungsgemäß hergestellten Oligonucleotidphosphonate weisen die folgende allgemeine Formel I auf:

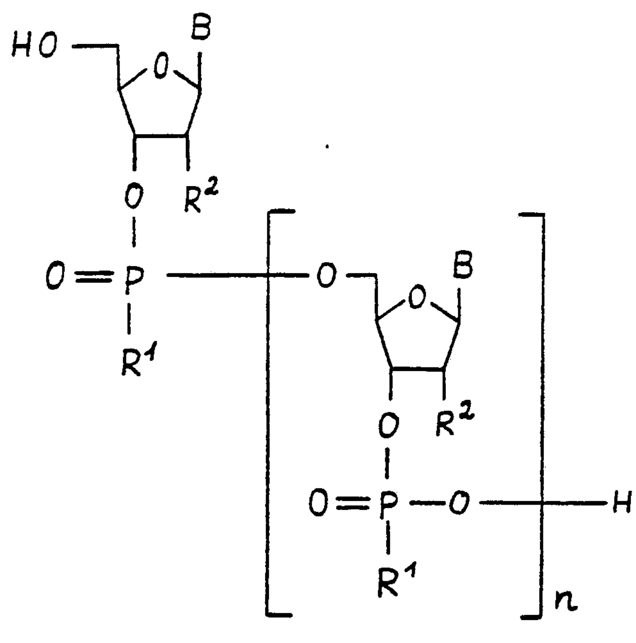

(I)

In der Formel I bedeutet

B eine Nucleobase, z. B. A, C, G oder T oder deren Analoga.

$R^1$ bedeutet eine gegebenenfalls verzweigte Alkylgruppe mit 1 - 10 Kohlenstoffatomen, die mit bis zu 4 Chloratomen oder einer Cyangruppe substituiert sein kann. Hierzu gehören u.a. Methyl, Ethyl, n-Propyl, i-Propyl, n-Butyl, sec.-Butyl, tert.-Butyl, 2,2,2-Trichlorethyl oder Cyanethyl. Die Gruppe $R^1$ kann weiterhin eine gegebenenfalls mit niedrigem Alkyl, niedrigem Alkoxy, Cyan, Halogen, insbesondere Chlor oder Brom, oder Nitro substituierte Aryl oder Aralkylgruppe mit 1 - 2 C-Atomen im Alkylteil sein, insbesondere eine Phenyl- oder Benzylgruppe.

Die Gruppe $R^2$ kann Wasserstoff sein; es handelt sich dann bei den Verbindungen der Erfindung um Oligodesoxynucleosidphosphonate. Die Gruppe $R^2$ kann auch Hydroxyl oder gegebenenfalls mit in der Nucleotidchemie üblichen Schutzgruppen geschütztes Hydroxyl sein. Derartige Schutzgruppen sind z. B. Trityl, Monomethoxytrityl und Dimethoxytrityl; Acyl, z. B. Acetyl oder Benzoyl; Tetrahydropyranyl, Methoxytetrahydropyranyl, o-Nitrobenzyl sowie Silylether wie z. B. t-Butyldiphenylsilylether.

n bedeutet in der allgemeinen Formel I eine ganze Zahl von 2 - 50.

Das Verfahren der Erfindung ist durch die folgenden Reaktionsschritte gezeichnet:

a) Umsetzung eines Nucleosids der allgemeinen Formel II

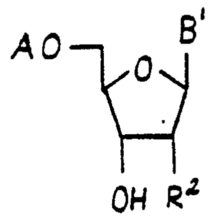

(II)

in der

A eine in der Nucleotidchemie übliche Schutzgruppe, die sich z. B. aus Tetrahedron 1981, Seite 363 - 369, Liebig's Ann. Chem. 1978, 839 - 850, sowie Nucleic Acids Research, Symposium Series No. 7, 1980, 39 - 59 ergibt, und

B' die gegebenenfalls mit in der Nucleotidchemie üblichen und ebenfalls in der oben genannten Literatur beschriebenen Schutzgruppen geschützte Nucleobase B bedeutet und

$R^1$ wie oben definiert ist,

mit einem Halogenphosphin der allgemeinen Formel III

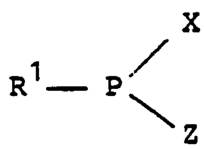

(III)

in der

X Chlor oder Brom,

Z Chlor oder Brom, $-NR^1_2$, wobei $R^1$ wie oben definiert ist, oder ein reaktiver heterocyclischer Rest ist (der reaktive heterocyclische Rest kann Triazolyl, Tetrazolyl, 3-Nitro-1,2,4-triazolyl oder dergleichen sein und ist über ein N-Atom mit dem P-Atom verbunden), und

$R^1$ wie oben definiert ist,

in Gegenwart einer organischen Base, z. B. Pyridin, Lutidin oder Collidin,

b) Umsetzung des in Schritt a erhaltenen Nucleosid-phosphonoderivates der allgemeinen Formel IV

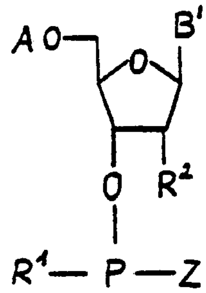

(IV)

in der A, B', $R^1$, $R^2$ und Z wie oben definiert sind,

mit einem an einen polymeren Träger gebundenen Nucleosid der allgemeinen Formel V

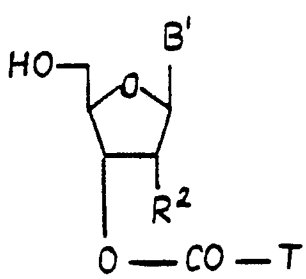

(V)

in der

B' und $R^2$ wie oben definiert und

T der polymere Träger ist, wobei als lösliche oder unlösliche (vernetzte) polymere Träger, z. B. modifiziertes Silicagel, Polyester, Polyamid, Polyvinylalkohol, Polysiloxan, Polystyrol, Glas oder dergleichen vorgesehen ist; als Verankerungsfunktion zwischen Träger und Nucleosid kommen vorzugsweise Esterbindungen in Betracht, auch solche, die sich vom Lävolinyl- oder β-Benzoylpropionylrest ableiten; die letztgenannten Esterbindungen können unter neutralen Bedingungen mit $N_2H_4$ gespalten werden,

c) Oxydation des in Stufe b erhaltenen an den Träger gebundenen Nucleotidonucleosids der allgemeinen Formel VI

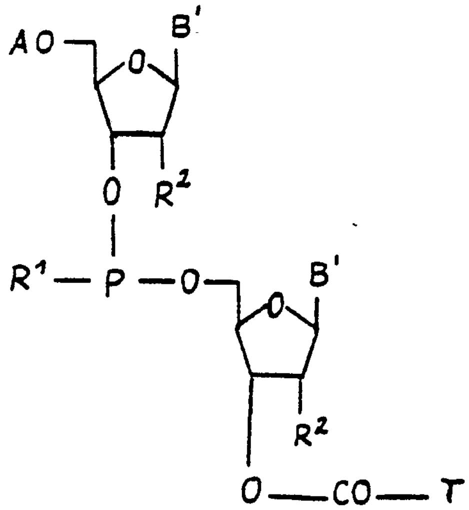

(VI)

in der R, B', $R^1$, $R^2$ und T wie oben definiert sind, wobei die Oxydation zum Phosphonat z. B. mit Jod erfolgen kann, allgemein unter Oxydation hier jedoch die Einführung von O, S oder Se zu verstehen ist,

d) Maskierung freier 5' OH-Gruppen die bei der Reaktion gemäß Stufe b nicht umgesetzt wurden, mit einer permanenten Schutzgruppe, z. B. durch Reaktion mit Acetanhydrid,

e) Abspaltung der Schutzgruppe A, z. B. durch Verwendung einer Protonsäure oder einer Lewis-Säure wie $ZnBr_2$ oder Dialkylaluminiumchlorid, wenn A eine Tritylgruppe oder ein Methoxyderivat derselben ist,

f) gegebenenfalls Einführung weiterer Nucleosidphosphonat- oder Oligonucleosidphosphonateinheiten entsprechend den Reaktionsschritten a - e sowie

g) Spaltung der Nucleosid-Trägerbindung mit Basen, z. B. Ammoniak, oder Hydrazin bei pH = 7, und gegebenenfalls Abspaltung aller in den Oligonucleosidphosphonaten, insbesondere den Nucleobasen, noch vorhandenen Schutzgruppen.

Die Zwischenprodukte der allgemeinen Formel IV stellen neue Verbindungen dar. Sie unterscheiden sich von den reaktionsträgen Phosphonigsäuremonoestern Z=O- durch eine um ein Vielfaches gesteigerte Reaktionsgeschwindigkeit bei dennoch gegebener Selektivität. Dadurch wird es möglich, die Verbindungen I in überraschend kurzer Zeit und mit unerwartet hohen Ausbeuten herzustellen, wenn man einen polymergebundenen Reaktanten gemäß der Formel V verwendet.

Die Erfindung wird im folgenden anhand eines Beispiels zur Herstellung der Deoxynucleosidmethylphosphondiester d(AC), d(AT), d(ATG) und d(ATTT) näher erläutert. Weitere Einzelheiten der Reaktion und physikalische Kenndaten der hergestellten Oligonucleosidphosphonate ergeben sich aus dem folgenden Reaktionsschema und der Tabelle.

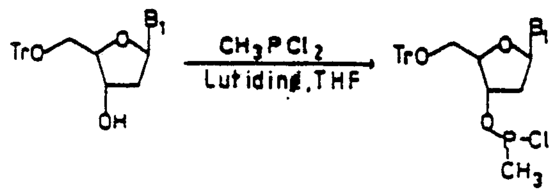

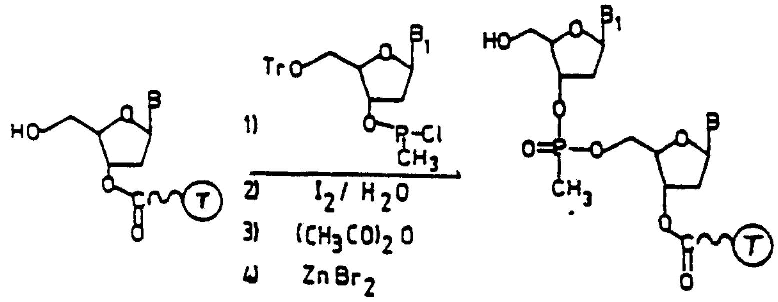


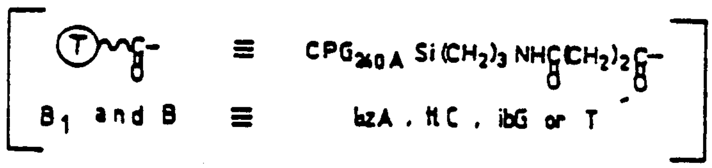


**Ausführungsbeispiel**

Das N-geschützte Nucleosid, gebunden an ein Glas mit gleichförmigen Porendurchmessern (CPG) wird wie in der Literatur beschrieben hergestellt, vgl. Tetrahedron Letters 1981, 4177.

Ein mMol N-Acyl-5'-O-trityldeoxynucleosid, gelöst in 1 ml trockenem THF, wird zu einem Gemisch von 5 mMol 2,6-Lutidin und 1 mMol Methylchlorphosphin, in 1 ml THF bei -78°C unter einer Schutzgasatmosphäre gegeben; die Zugabezeit beträgt 15 - 20 Minuten. Nach 30 Minuten läßt man das Gemisch langsam auf Raumtemperatur erwärmen. Das Lutidin-Hydrochlorid wird abfiltriert; das Filtrat wird unter verringertem Druck eingeengt. Man dampft zweimal mit Toluol/THF (10 ml, 1 : 1) bis zur Schaumbildung ein und löst das Gemisch in 5 ml THF, das 5 mMol 2,6-Lutidin enthält; man erhält eine 0,2 M-Lösung. Die aktive Nucleosidmethylphosphonomonochloriditlösung (1 ml) wird mit einer Spritze in eine mit einem Septum verschlossene Säule gegeben, die das an CPG-Perlen (100 - 200 mg) gebundene Nucleosid (10 - 20 µMol) enthält. Nach einer Reaktionszeit von 15 Minuten wird das Reagenz durch Schutzgasdruck entfernt. Das System wird evakuiert und erneut mit 1 ml des aktiven Nucleosids 10 Minuten beschickt. Die Kondensationsausbeute wird durch Bestimmung der Tritylkationenkonzentration bestimmt. Nach einer mehr als 95-%-igen Kondensation wird der Phosphitdiester mit 0,1 M Jod in THF/Pyridin/Wasser (40 : 20 : 1) oxidiert; die Oxidation ist nach 3 bis 5 Minuten beendet. Überschüssiges Jod wird durch Waschen mit Pyridin (5 - 10 ml) bis zum Ausbleiben der Farbe entfernt; danach wird mit THF (20 - 30 ml) gewaschen. 2 ml eines Gemisches von DMAP/THF/$Ac_2O$/Lutidin (0,6 g, 10 ml, 1,1 g) wird durch die Säule geleitet, wonach man mit 5 ml Pyridin und anschließend mit 30 - 50 ml THF wäscht, um gegebenenfalls zurückgebliebenes Pyridin und Lutidin zu entfernen. Die Kolonne wird im Hochvakuum 10 Minuten lang getrocknet. Eine gesättigte Lösung von $ZnBr_2$ entweder in $CH_2Cl_2$/MeOH (7 : 3) oder Nitromethan/Wasser (100 : 1) wird durch die Perlen geleitet, bis die Detritylierung beendet ist. Anschließend wird mit nBuOH/Lutidin/THF (4/1 : 5) (5 ml) gewaschen; schließlich wird nochmals allein mit 30 - 50 ml THF gewaschen. Anschließend wird die nächste Nucleosideinheit eingeführt, indem man das obige Verfahren mit dem geeigneten Nucleosid-methylphosphomonochloridit wiederholt. Die Zeit für einen vollständigen Reaktionscyclus beträgt 45 - 60 Minuten, im Gegensatz von 4 - 6 Stunden bei Verwendung eines Phosphonomonoesters und MSNT des Kondensationsmittels.

Die Nucleosid-methylphosphonodiesterkette wird dann von dem Träger mit Ammoniak (25 %, 2 ml) bei 35°C und einer Reaktionszeit von 15 Stunden entfernt. Die überstehende Flüssigkeit wird entfernt; die Perlen werden mit bidestilliertem Wasser (2,3 ml) gespült. Die vereinigten Flüssigkeiten werden unter verringertem Druck eingeengt und an Silicagel-Platten (Acetonitril/Wasser = 80 : 20) dünnschichtchromatographisch zur Entfernung von nichtnucleotidischem Material entfernt. Die Nucleotidbande auf der Platte wird mit $CHCl_3$/MeOH (7 : 3) eluiert. Das Produkt wird konzentriert und mit bidestilliertem Wasser lyophilisiert.

In gleicher Weise können sowohl Purin als auch Pyrimidin-deoxynucleosid-methylphosphonomonochloridite für die Kondensation mit CPG-gebundenen Purin- und Pyrimidin-deoxynucleosiden eingesetzt werden. Auf diese Weise wurden d(AC), d(AT), d(ATG) und d(ATTT)-methylphosphonodiester hergestellt. Die Gesamtausbeuten, UV-Absorptionsmaxima und Rf-Werte ergeben sich aus der Tabelle.

| Oligomer | Ausbeute (%) | λmax (nm)[d] | λmin(nm)[d] | Rf a) | Rf b) |
|---|---|---|---|---|---|
| d(AC) | 93 | 265 | 245 | 0,12[c] | 0,04[c] |
| d(AT) | 94 | 264 | 234 | 0,26[c] | 0,05[c] |
| d(ATG) | 85 | 271 | 243 | 0,21 | 0 |
| d(ATTT) | 76 | 268 | 243 | 0,32 | 0 |

a) $CH_3CN/H_2O = 8:2$ (v/v);
b) $CH_3OH/CHCl_3 = 7:3$ (v/v),
c) Es wurden 2 Diastereomere beobachtet, die sich jedoch nicht als getrennte Flecken auftrennten;
d) Gemessen in 10 mM Tris·HCl (pH 7,5), 1 mM EDTA.

**Patentanspruch**

Verfahren zur Herstellung von Oligonucleosidphosphonaten der allgemeinen Formel I

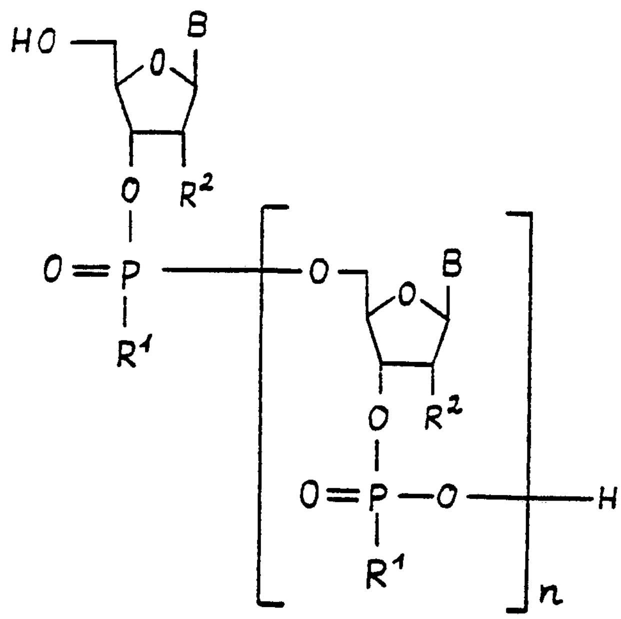

(I)

in der
B eine Nucleobase,
$R^1$ eine gegebenenfalls verzweigte Alkylgruppe mit 1 - 10 C-Atomen, die mit bis zu 4 Chloratomen oder einer Cyangruppe substituiert sein kann, oder eine gegebenenfalls mit niedrigem Alkyl, niedrigem Alkoxy, Cyan, Halogen oder Nitro substituierte Aryl- oder Aralkylgruppe mit 1 - 2 C-Atomen im Alkylteil,
$R^2$ Wasserstoff, Hydroxyl oder gegebenenfalls mit in der Nucleotidchemie üblichen Schutzgruppen geschütztes Hydroxyl und
n eine ganze Zahl von 2 - 50 bedeuten,
gekennzeichnet durch die folgenden Reaktionsschritte:
a) Umsetzung eines Nucleosids der allgemeinen Formel II

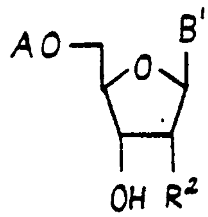

(II)

in der
A eine in der Nucleotidchemie übliche Schutzgruppe und
B' die gegebenenfalls mit in der Nucleotidchemie üblichen Schutzgruppen geschützte Nucleobase B bedeuten und

$R^2$ wie oben definiert ist,
mit einem Halogenphosphin der allgemeinen Formel III

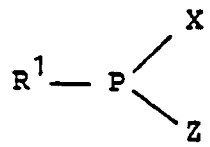

(III)

in der
X Chlor oder Brom,
Z Chlor oder Brom, - $NR^1_2$ wobei $R^1$ wie oben definiert ist, oder ein reaktiver heterocyclischer Rest ist,
in Gegenwart einer organischen Base,
b) Umsetzung des in Schritt a erhaltenen Nucleosid-phosphonomonoderivates der allgemeinen Formel IV

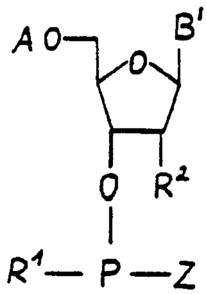

(IV)

in der A, B', $R^1$, $R^2$ und Z wie oben definiert sind, mit einem an einen polymeren Träger gebundenen Nucleosid der allgemeinen Formel V

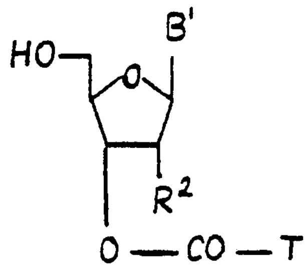

(V)

in der
B' und $R^2$ wie oben definiert sind und
T der polymere Träger ist,
c) Oxidation des in Stufe b erhaltenen trägergebundenen Nucleotidonucleosids der allgemeinen Formel VI

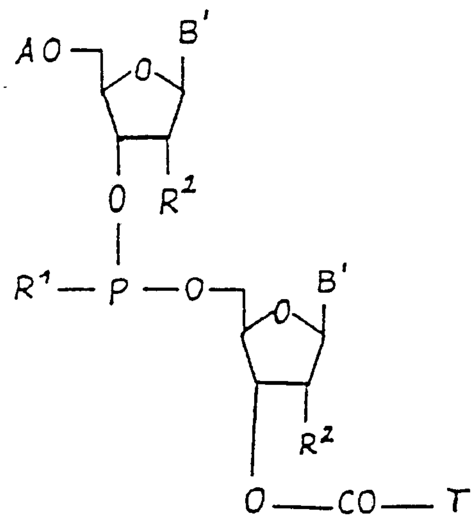

(VI)

in der A, B', $R^1$, $R^2$ und T wie oben definiert sind, wobei die Oxydation zum Phosphonat z. B. mit Jod erfolgen kann, allgemein unter Oxydation hier jedoch die Einführung von O, S oder Se zu verstehen ist,

d) Maskierung freier 5' OH-Gruppen die bei der Reaktion gemäß Stufe b nicht umgesetzt wurden, mit einer permanenten Schutzgruppe, z. B. durch Reaktion mit Acetanhydrid oder

e) Abspaltung der Schutzgruppe A, z. B. durch Verwendung einer Protonsäure oder einer Lewis-Säure wie $ZnBr_2$ oder Dialkylaluminiumchlorid, wenn A eine Tritylgruppe oder ein Methoxyderivat derselben ist,

f) gegebenenfalls Einführung weiterer Nucleosidphosphonat- oder Oligonucleosidphosphonateinheiten entsprechend den Reaktionsschritten a - e sowie

g) Spaltung der Nucleosid-Trägerbindung mit Basen, z. B. Ammoniak, oder Hydrazin bei pH = 7, und gegebenenfalls Abspaltung aller in den Oligonucleosidophosphonaten, insbesondere den Nucleobasen, noch vorhandenen Schutzgruppen.

**Revendication**

1. Procédé de préparation d'oligophosphonates de nucléosides de formule générale I

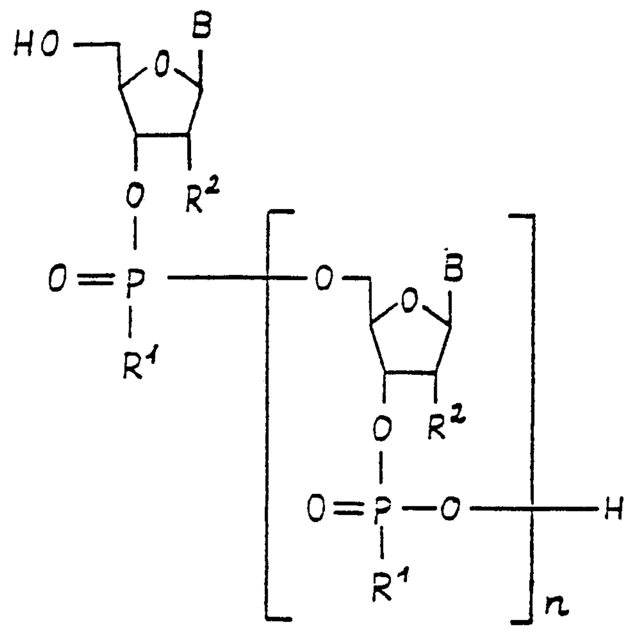

(I)

dans laquelle

B représente une nucléobase,

$R^1$ représente un groupe alkyle éventuellement ramifié en $C_1$ - $C_{10}$, qui peut porter jusqu'à 4 atomes de chlore ou un groupe cyano en tant que substituants, ou un groupe aryle ou aralkyle contenant 1 à 2 atomes de carbone dans la partie alkyle, éventuellement substitué par un groupe alkyle inférieur, alcoxy inférieur, cyano, halogéno ou nitro,

$R^2$ représente l'hydrogène, un groupe hydroxy ou un groupe hydroxy éventuellement protégé par des groupes protecteurs usuels de la chimie des nucléotides, et

n est un nombre entier qui vaut de 2 à 50, caractérisé par les étapes de réaction suivantes:

a) réaction d'un nucléoside de formule générale II

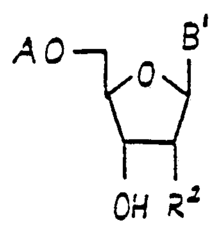

(II)

dans laquelle

A représente un groupe protecteur usuel de la chimie des nucléotides et

B' la nucléobase B, éventuellement protégée par des groupes protecteurs usuels dans la chimie des nucléotides, et

$R^2$ a les significations indiquées ci-dessus,

avec une halogénophosphine de formule générale III

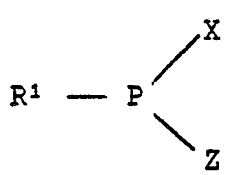

(III)

dans laquelle

X représente le chlore ou le brome,

Z représente le chlore ou le brome, un groupe $-NR^1_2$ dans lequel le $R^1$ a les significations indiquées ci-dessus, ou un radical hétérocyclique réactif,

en présence d'une base organique,

b) réaction du dérivé monophosphononucléoside obtenu dans l'étape a), répondant à la formule générale VI

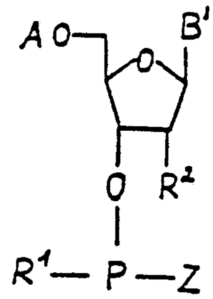

(IV)

dans laquelle A, B', $R^1$, $R^2$ et Z ont les significations indiquées ci-dessus, avec un nucléoside fixé sur un support polymère et répondant à la formule générale V

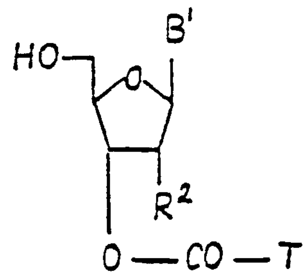

(V)

dans laquelle

B' et $R^2$ ont les significations indiquées ci-dessus et

T représente le support polymère,

c) oxydation du nucléotidonucléoside fixé sur support obtenu dans l'étape b), répondant à la formule générale VI

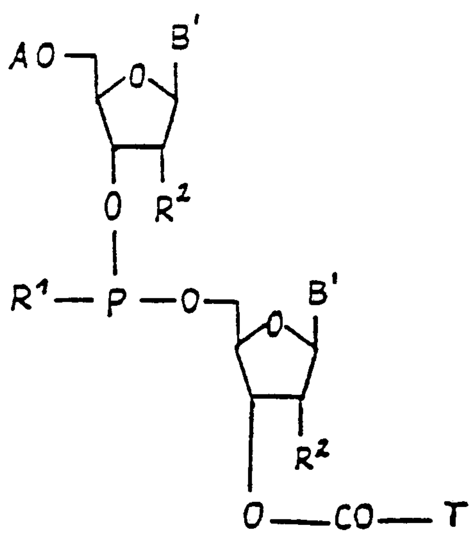

(VI)

dans laquelle A, B', $R^1$ et T ont les significations indiquées ci-dessus, l'oxydation en le phosphonate pouvant être réalisée par exemple à l'aide d'iode, étant entendu que l'oxydation dont il est question ici consiste d'une manière générale en l'introduction de O, S ou Se,

d) blocage des groupes 5'-OH libres qui n'ont pas réagi lors de la réaction de l'étape b), par un groupe protecteur permanent, par exemple par réaction avec l'anhydride acétique, ou bien

e) élimination du groupe protecteur A, par exemple à l'aide d'un acide protonique ou d'un acide de Lewis tel que $ZnBr_2$ ou un chlorure de dialkyl-aluminium, lorsque A représente un groupe trityle ou un dérivé méthoxylé de celui-ci,

f) le cas échéant, introduction d'autres motifs phosphonates de nucléosides ou oligophosphonates de

nucléosides par les étapes de réaction a) à e), et

g) scission de la liaison nucléoside-support à l'aide de bases, par exemple l'ammoniac, ou l'hydrazine à pH 7, et éventuellement élimination de tous les groupes protecteurs encore présents dans les oligophosphonates de nucléosides, en particulier dans les nucléobases.

**Claim**

Process for the preparation of oligonucleoside phosphonates of the general formula (I)

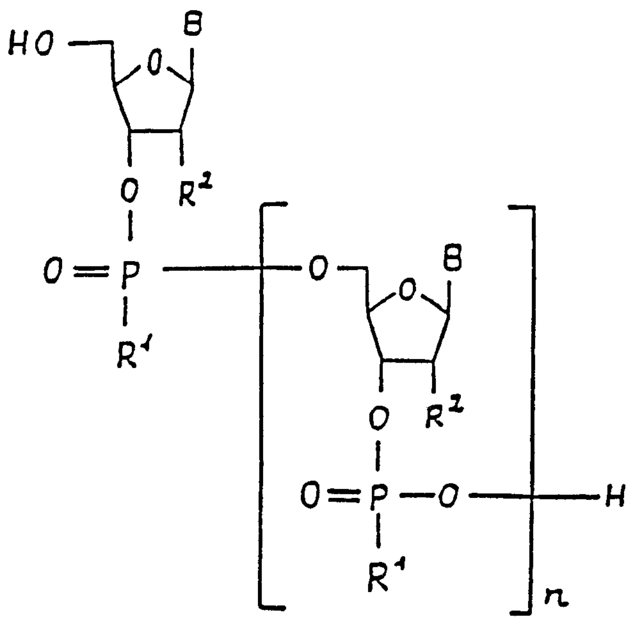

(I)

in which

B denotes a nucleo-base,

$R^1$ denotes an optionally branched alkyl group with 1 - 10 C atoms, which can be substituted by up to 4 chlorine atoms or by a cyano group, or an aryl or aralkyl group which has 1 - 2 C atoms in the alkyl part and is optionally substituted by lower alkyl, lower alkoxy, cyano, halogen or nitro,

$R^1$ denotes hydrogen, hydroxyl or hydroxyl which is protected, if appropriate, with the protective groups customary in nucleotide chemistry and

n denotes an integer from 2 to 50,

characterized by the following reaction steps:

a) reaction of a nucleoside of the general formula (II)

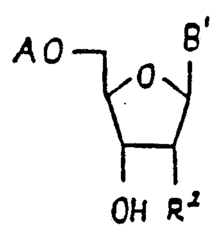

(II)

in which

A is a conventional protective group in nucleotide chemistry,

B' is the nucleo-base B, which is optionally protected by conventional protective groups in nucleotide chemistry and

$R^2$ is as defined above,

with a halogenophosphine of the general formula (III)

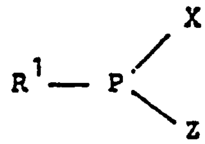

(III)

in which

X is chlorine or bromine,

Z is chlorine or bromine, $-NR^1_2$, $R^1$ being defined as hereinbefore or a reactive heterocyclic radical, in the presence of an organic base,

b) reaction of the monophosphono-nucleoside derivative obtained in step a), of the general formula (IV)

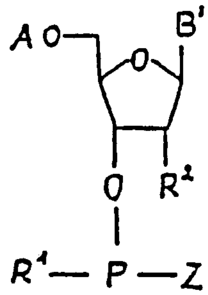

(IV)

in which A, B', $R^1$, $R^2$ and Z are as defined above, with a nucleoside bound to a polymeric carrier, of the general formula (V)

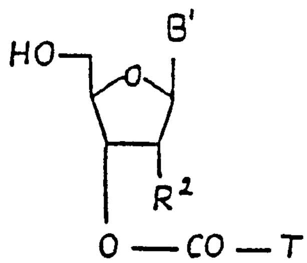

(V)

in which

B' and $R^2$ are as defined above and

T is the polymeric carrier,

c) oxidation of the carrier-bound nucleotidonucleoside obtained in stage b), of the general formula (VI)

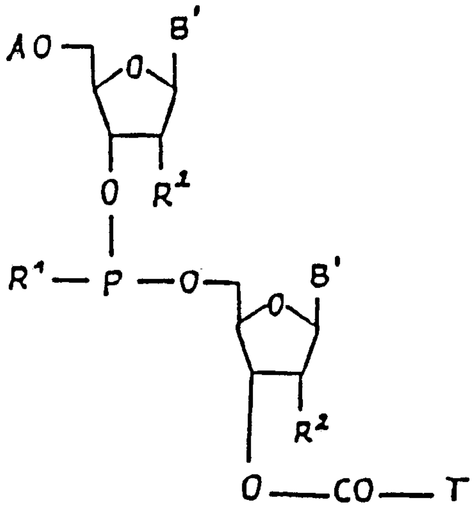

(VI)

in which A, B', $R^1$, $R^2$ and T are as defined above, whereby the oxidation to the phosphonate can e.g. be carried out with iodine, but oxidation here is generally understood as the introduction of O, S or Se,

d) masking of free 5'-OH groups, which have not been reacted in the reaction according to stage b), with a permanent protective group, e.g. by reaction with acetic anhydride,

e) splitting off of the protective group A, e.g. using a protic acid or a Lewis acid such as $ZnBr_2$ or dialkyl aluminium chloride, if A is a trityl group or a methoxy derivative thereof,

f) if appropriate introduction of further nucleoside phosphonate or oligonucleoside phosphonate units in accordance with reactions a) - e), and

g) splitting off of the nucleoside-carrier bond with bases, e.g. ammonia or hydrazine at pH = 7 and, if appropriate, splitting off all the protective groups present in the oligonucleoside phosphonates obtained, particularly the nucleo-bases.

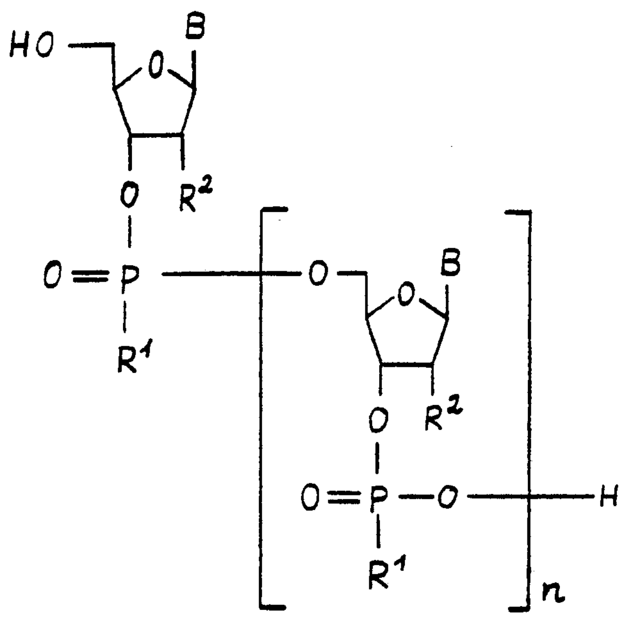

(I)

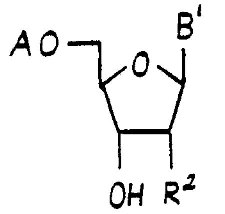

(II)

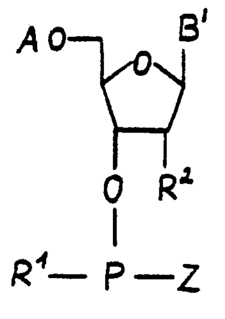

(III)

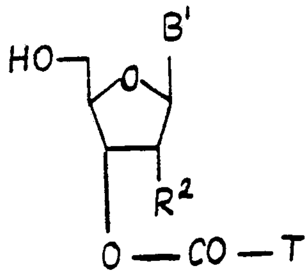

(IV)

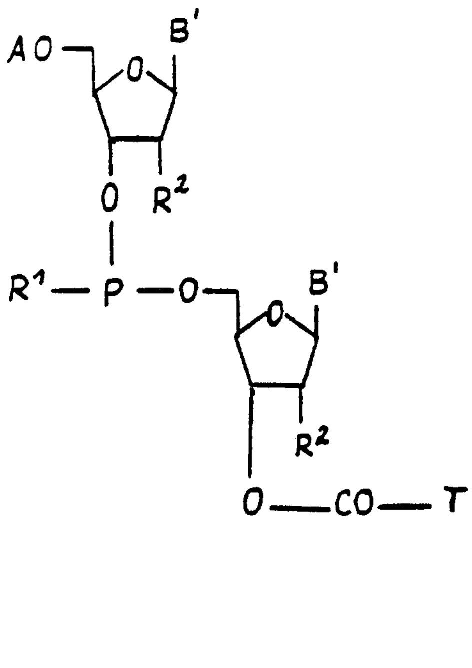

(V)